**Europäisches Patentamt**

**European Patent Office**

**Office européen des brevets**

(19)

(11) Veröffentlichungsnummer: **0 156 266**

**A2**

(12) # EUROPÄISCHE PATENTANMELDUNG

(21) Anmeldenummer: 85103021.3

(22) Anmeldetag: 15.03.85

(51) Int. Cl.⁴: **C 07 K 15/06**
**C 07 K 15/14, C 07 K 3/18**
**G 01 N 33/68**

(30) Priorität: 23.03.84 DE 3410694

(43) Veröffentlichungstag der Anmeldung:
02.10.85 Patentblatt 85/40

(84) Benannte Vertragsstaaten:
AT BE CH DE FR GB IT LI LU NL SE

(71) Anmelder: **BEHRINGWERKE AKTIENGESELLSCHAFT**
**Postfach 1140**
**D-3550 Marburg/Lahn(DE)**

(72) Erfinder: **Bohn, Hans, Dr.**
**Oberer Eichweg 26**
**D-3550 Marburg(DE)**

(72) Erfinder: **Winckler, Wilhelm**
**Untere Bergstrasse 21**
**D-3556 Wenkbach(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Gewebeprotein PP21, Verfahren zu seiner Gewinnung sowie seine Verwendung.

(57) Es wird das Protein $PP_{21}$ beschrieben, das

a) eine elektrophoretische Beweglichkeit im Bereich der von $ß_1$-Globulinen;

b) einen isoelektrischen Punkt von 4,6 ± 0,3;

c) einen Sedimentationskoeffizienten $s_{20,w}$ von 3,2 ± 0,2 S;

d) ein in einer Ultrazentrifuge bestimmtes Molekulargewicht von 52.900 ± 6.200;

e) einen Extinktionskoeffizienten $E_{1\%\ 1cm}$(280 nm) von 10,5 ± 1,0; und

f) einen Kohlenhydratanteil von 19,2 ± 5,2 g/100 g (Mannose 1,8 ± 0,4, Galactose 4,3 ± 1,0, Fucose 1,3 ± 0,3, N-Acetyl-Glukosamin 6,5 ± 2,0, N-Acetyl-Neuraminsäure 5,3 ± 1,5, jeweils g/100 g)

und eine bestimmte Aminosäurezusammensetzung hat, ein Verfahren zu seiner Gewinnung sowie seine Verwendung.

0156266

BEHRINGWERKE AKTIENGESELLSCHAFT    84/B 003 -

Dr. Ha/Sd.

Gewebeprotein $PP_{21}$, Verfahren zu seiner Gewinnung sowie
seine Verwendung

---

Die Erfindung betrifft ein Gewebeprotein, das als $PP_{21}$
bezeichnet wird, sowie ein Verfahren zu seiner Gewinnung. $PP_{21}$ kann verwendet werden, um Antiseren herzustellen, die dazu dienen können, $PP_{21}$ im Gewebe und in
Körperflüssigkeiten nachzuweisen und zu bestimmen. Der
Nachweis und die Bestimmung von $PP_{21}$ haben diagnostische
Bedeutung. Sie können dazu dienen, Erkrankungen bestimmter Organe zu erkennen, einen Krankheitsverlauf zu überwachen oder eine Therapie zu kontrollieren.

Proteine sind zwar im Stand der Technik bekannt, auch
Gewebeproteine, jedoch keines mit den nachfolgend für
$PP_{21}$ angegebenen Eigenschaften.

Gegenstand der Erfindung ist das Protein $PP_{21}$, gekennzeichnet durch

a) eine elektrophoretische Beweglichkeit im Bereich der
   von $\beta_1$-Globulinen;

b) einen isoelektrischen Punkt von $4,6 \pm 0,3$;

c) einen Sedimentationskoeffizienten $s_{20,w}$ von $3,2 \pm 0,2$ S;

d) ein in einer Ultrazentrifuge bestimmtes Molekulargewicht von $52.900 \pm 6.200$;

e) einen Extinktionskoeffizienten $E_{1cm}^{1\%}$(280 nm) von $10,5 \pm 1,0$; und

f) einen Kohlenhydratanteil von $19,2 \pm 5,2$ g/100 g
   (Mannose $1,8 \pm 0,4$, Galactose $4,3 \pm 1,0$, Fucose $1,3 \pm 0,3$, N-Acetyl-Glukosamin $6,5 \pm 2,0$, N-Acetyl-Neuraminsäure $5,3 \pm 1,5$, jeweils g/100 g).

- 2 -

Die Aminosäurenzusammensetzung von $PP_{21}$ ist in der folgenden Tabelle gegeben:

| Aminosäure | Reste pro 100 Reste | Variations-koeffizient |
|---|---|---|
| Lysin | 8,75 | 3,91 |
| Histidin | 1,58 | 6,81 |
| Arginin | 2,75 | 6,07 |
| Asparaginsäure | 9,59 | 1,97 |
| Threonin | 3,73 | 7,22 |
| Serin | 7,43 | 5,95 |
| Glutaminsäure | 8,16 | 5,15 |
| Prolin | 11,34 | 9,36 |
| Glycin | 7,01 | 1,25 |
| Alanin | 4,98 | 3,62 |
| Cystin 1/2 | 5,50 | 0,84 |
| Valin | 8,14 | 1,42 |
| Methionin | 0,00 | 0,00 |
| Isoleucin | 4,86 | 2,78 |
| Leucin | 5,42 | 10,34 |
| Tyrosin | 5,01 | 5,01 |
| Phenylalanin | 3,56 | 1,00 |
| Tryptophan | 2,12 | 17,65 |

Zur Erläuterung der kennzeichnenden Merkmale des Gewebsproteins sei folgendes ausgeführt:

Die elektrophoretische Beweglichkeit wurde in der Mikromodifikation mit dem Gerät Microzone R 200 von Beckman Instruments auf Zelluloseazetatfolien (Firma Sartorius) unter Verwendung von Natriumdiethylbarbiturat-Puffer, pH 8,6, bestimmt.

Der isoelektrische Punkt wurde mit einer Säule (440 ml) der Firma LKB, Stockholm, ermittelt. Das Ampholin®-Gemisch hatte einen pH-Bereich von 4,0 bis 6,0.

Der Sedimentationskoeffizient wurde in einer analytischen Ultrazentrifuge der Firma Beckman (Spinco-Apparatur, Modell E) bei 60.000 UpM in Doppelsektorzellen mit Hilfe der UV-Scanner-Technik bei 280 nm bestimmt. Als Lösungsmittel diente Wasser. Die Proteinkonzentration betrug 0,2 g/100 ml.

Zur Ermittlung des Molekulargewichts in der Ultrazentrifuge wurde die Sedimentationsgleichgewichtsmethode herangezogen. Die Konzentration des Proteins ist dabei auf etwa 1,0 O.D. (optische Dichte) eingestellt worden. Als Lösungsmittel diente ein 0,05 mol/l Phosphatpuffer (pH 6,8) der 0,2 mol/l NaCl enthielt. Die Bestimmung wurde bei 9.000 UpM vorgenommen. Die Registrierung erfolgte mit einer UV-Optik bei 280 nm unter Einsatz eines photoelektrischen Scanners.

Zur Bestimmung des Extinktionskoeffizienten wurde die Substanz zu einer Konzentration von 1 g/l in destilliertem Wasser gelöst.

Die Analyse der Kohlenhydrate wurde wie folgt durchgeführt: Nach Hydrolyse der glykosidischen Bindungen wurden die freigesetzten Neutralzucker als Boratkomplexe über eine Anionenaustauschersäule getrennt (Y.C. Lee et al., Anal.Biochem. (1969), 27, 567), im Eluat durch Zumischen von Cu(I)-bicinchoninatreagenz (K. Mopper und M. Gindler, Anal.Biochem. (1973), 56, 440) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, (1963), Vol.VI, 463-465) ermittelt worden.

Die Aminosäurenanalyse wurde nach S. Moore et al., Anal. Chem. (1958), $\underline{30}$, 1185, unter Verwendung eines Flüssigkeitschromatographen Multichrom B der Firma Beckman durchgeführt. 1/2 Cystin wurde nach Oxydation der Proteine mit Perameisensäure (S. Moore et al., Anal. Chem. (1958), $\underline{30}$, 1185) und nachfolgender Chromatographie (S. Moore, J.Biol.Chem. (1963), $\underline{238}$, 235) als Cysteinsäure bestimmt. Der Tryptophangehalt ist mit der direkten photometrischen Bestimmung nach B. Edelhoch, Biochemistry (1967), $\underline{6}$, 1948, ermittelt worden.

Bei der Untersuchung von Extrakten aus verschiedenen menschlichen Organen konnte $PP_{21}$ in der Plazenta, im Magen, im Darmtrakt (Colon, Jejunum), in der Blase sowie in der Milz nachgewiesen werden. Extrakte aus anderen Organen des Menschen wie Herz, Lunge, Leber, Haut, Niere, Nebenniere, und Uterus enthielten dieses Protein nicht oder nur in Spuren.

Zur Isolierung von $PP_{21}$ können Organe des Menschen, in denen dieses Protein vorkommt, verwendet werden. Insbesondere eignen sich dafür ausgewachsene menschliche Plazenten, die in reichlicher Menge anfallen und das Protein in genügend großer Konzentration enthalten. Aus einer ausgewachsenen menschlichen Plazenta (600g) lassen sich mit physiologischen Salzlösungen im Durchschnitt 7 mg $PP_{21}$ extrahieren.

Ein sich immunchemisch wie $PP_{21}$ verhaltendes Antigen ist außerdem Bestandteil der sogenannten $MP_2$-Proteine der menschlichen Plazenta. Die $MP_2$-Proteine sind eine Gruppe komplex zusammengesetzter Gewebeproteine, die offensichtlich in der Plazenta mit Membranen assoziiert sind. Sie werden aus dem nach Waschen mit physiologischer Salzlösung unlöslichen Geweberückstand der Plazenta durch Behandlung mit solubilisierenden Agentien zum Beispiel mit dem nicht-ionischen Detergens Triton® X-100 erhalten.

Ihre Isolierung und Charakterisierung wurde in der deutschen Patentanmeldung 33 34 405, angemeldet am 23.9.83, beschrieben. Die $MP_2$-Proteine haben Molekulargewichte zwischen 200.000 und mehr als 1.000.000. An ihrem Aufbau sind insgesamt mindestens vier verschiedene Komponenten beteiligt, die sich in ihren Antigendeterminanten unterscheiden. Eine dieser, als C bezeichneten, Komponenten ist immunchemisch ununterscheidbar von dem in der vorliegende Anmeldung beschriebenen löslichen Gewebeprotein $PP_{21}$.

$PP_{21}$ hat folgende Eigenschaften, die sich in einem Verfahren zu seiner Isolierung verwenden lassen, indem diesen Eigenschaften entsprechende Maßnahmen getroffen werden:

1) Mit Ammoniumsulfat wird es bei einem pH von 6-8 und 30-60% Sättigung aus wässrigen Lösungen gefällt;

2) Mit wasserlöslichen Acridinbasen, beispielsweise 2-Ethoxi-6,9-diaminoacridinlactat wird es bei pH-Werten zwischen 7 und 9 und einer Konzentration der Base von 4 bis 8 g/l präzipitiert, nicht oder kaum dagegen bei pH 6,0, wenn die Konzentration der Base 4 g/l oder kleiner ist;

3) Bei Zugabe von Ethanol zu verdünnten Salzlösungen, zum Beispiel 10-20 g/l NaCl-Lösung, in denen es gelöst ist, bleibt es bei pH 7 bis zu einer Konzentration von 200 ml Alkohol/l großenteils im Überstand;

4) Bei einer elektrophoretischen Auftrennung wird es bei pH 7-9 im Bereich der $\beta_1$-Globuline gefunden;

5) Bei der isoelektrischen Fokussierung erscheint es im pH-Bereich von 4,3 bis 4,9 mit der Hauptmenge zwischen 4,4 und 4,8;

6) Bei der Gelfiltration mit Sephadex® verhält es sich wie Proteine mit Molekulargewichten von 40.000 bis 100.000;

7) Es läßt sich an schwach basische Ionenaustauscher, beispielsweise DEAE-Cellulose oder DEAE-Sephadex®, bei einer Leitfähigkeit von etwa 0-2 mS und einem pH-Wert von etwa pH 7 bis 9 binden und kann mit stärker konzentrierten Salzlösungen, beispielsweise 10-50 g/l NaCl-Lösungen, eluiert werden;

8) Es kann aus einer wässrigen Lösung durch Immunadsorption angereichert und isoliert werden.

Gegenstand der Erfindung ist demnach auch ein Verfahren zur Gewinnung oder Anreicherung des $PP_{21}$, dadurch gekennzeichnet, daß ein aus Organen, die dieses Protein enthalten, mit einer verdünnten Salz- oder Pufferlösung gewonnener Extrakt einer oder mehreren der folgenden Maßnahmen unterworfen wird:

a) Fällung des Proteins $PP_{21}$ mit Ammoniumsulfat im pH-Bereich von 6 bis 8 und bei 30-60% Sättigung;

b) Abscheidung von Begleitproteinen mit einer wasserlöslichen Acridinbase bei einem pH-Wert von 6 und einer Konzentration der Base von 4 g/l oder weniger oder Fällung des Proteins $PP_{21}$ mit einer wasserlöslichen Acridinbase bei einem pH-Wert zwischen 7 und 9 und einer Konzentration der Base von 4 bis 8 g/l;

c) Abscheidung von Begleitproteinen durch Zugabe von bis zu 200 ml Ethanol/l bei pH 7;

d) präparative Zonenelektrophorese, wobei die Proteinfraktion mit einer Beweglichkeit im Bereich der $\beta_1$-Globuline gewonnen wird;

e) isoelektrische Fokussierung, wobei die Proteine im pH-Bereich von 4,4 - 4,8 gewonnen werden;

f) Gelfiltration oder Ultrafiltration, wobei die Proteine im Molekulargewichtsbereich von 40.000 bis 100.000 gewonnen werden;

g) Adsorption an einem schwach basischen Ionenaustauscher und Elution des Proteins $PP_{21}$;

h) immunadsorptive Anreicherung.

Neben Ammoniumsulfat können selbstverständlich auch andere in der präparativen Biochemie üblicherweise einge-

setze Neutralsalze zur Ausfällung des $PP_{21}$ verwendet werden. Neben einer Acridinbase ist auch ein wasserlösliches Derivat einer Chinolinbase, wie sie für Proteinfraktionierungen bekannt sind, im Rahmen des erfindungsgemäßen Verfahrens einsetzbar. Entsprechend seinem elektrophoretischen Verhalten, seinem isoelektrischen Punkt oder seinem Molekulargewicht, sind zur Isolierung des Proteins auch andere Maßnahmen brauchbar, die geeignet sind, ein Protein mit den angegebenen Eigenschaften von anderen Proteinen abzutrennen. Hierzu können die verschiedenen Methoden der präparativen Elektrophorese, der isoelektrischen Fokussierung, der Gelfiltration, Gelchromatographie oder Ultrafiltration oder auch die Eigenschaft des $PP_{21}$, an schwach basische Ionenaustauscher gebunden und hiervon wieder eluiert werden zu können, verwendet werden.

Durch eine zweckmäßige Kombination der genannten Maßnahmen, die eine Anreicherung des $PP_{21}$ oder eine Abtrennung dieses Proteins von anderen Proteinen bewirken, kann das $PP_{21}$ isoliert werden.

Demzufolge ist ein Gegenstand der vorliegenden Erfindung in den einzelnen Schritten zur Anreicherung des $PP_{21}$ und in dem durch Kombination der Maßnahmen zur Anreicherung sich ergebenden Verfahren zur Reinigung des $PP_{21}$ zu sehen.

Die im Beispiel angegebenen Schritte zur Anreicherung und Isolierung von $PP_{21}$ sind keinesfalls alle zwingend und müssen auch nicht in der dort beschriebenen Reihenfolge durchgeführt werden.

Zur Immunadsorption könnte man direkt den Extrakt aus menschlichen Plazenten einsetzen. Da die Konzentration von $PP_{21}$ im Plazentenextrakt aber relativ gering ist, ist es zweckmäßig, durch eine Vorfraktionierung es Extraktes das Protein $PP_{21}$ erst spezifisch anzureichern mit Hilfe von Methoden, die sich zur Fraktionierung von Proteinen in größerem Maßstab eignen; zum Beispiel durch fraktionierte Fällung mit Neutralsalzen oder organischen Kationen, durch Gelfiltration oder durch Ionenaustauschchromatographie. Auch der Schritt der Immunadsorption könnte durch Anwendung anderer Trennmethoden, zum Beispiel durch präparative Elektrophorese oder isoelektrische Fokussierung, ersetzt werden.

Zur Hochreinigung von $PP_{21}$ im Endstadium der Isolierung haben sich Gelfiltration an Ultrogel® AcA 34 und inverse Immunadsorption als brauchbar erwiesen.

Zum Nachweis und zur Bestimmung des $PP_{21}$, etwa in einer Fraktion aus einer Trennoperation, können neben den angegebenen Parametern auch immunchemische Methoden dienen, da $PP_{21}$ antigene Eigenschaften hat.

Ein zum immunchemischen Nachweis von $PP_{21}$ geeignetes Antiserum wurde erstmals durch Immunisierung von Kaninchen mit den membranassoziierten Proteinen $MP_2$ der Plazenta gewonnen. Die dabei erhaltenen Antiseren enthielten Antikörper gegen mindestens vier unterschiedliche Antigenkomponenten (A, B, C und D) in den $MP_2$-Proteinen. Es wurde gefunden, daß die gegen die Komponente C gerichteten Antikörper auch mit einem löslichen Protein der Plazenta präzipitieren, nämlich mit dem in der vorliegenden Anmeldung beschriebenen Gewebeprotein $PP_{21}$.

Die Anti-$MP_2$ Kaninchenseren ließen sich einerseits zum immunlogischen Nachweis von $PP_{21}$ in löslichen Proteinfraktionen der Plazenta, andererseits zur Herstellung eines Immunadsorbens zur Anreicherung und Isolierung von $PP_{21}$ aus löslichen Plazentaproteinfraktionen einsetzen.

Dies war möglich, da die anderen am Aufbau der MP$_2$-Proteine beteiligten Antigenkomponenten in den löslichen Plazentaproteinfraktionen nicht oder höchstens in Spuren vorkommen.

Mit Hilfe des nach der vorliegenden Anmeldung gewonnenen gereinigten PP$_{21}$ lassen sich durch Immunisieren von Tieren nach bekannten Methoden monospezifische Antiseren herstellen.

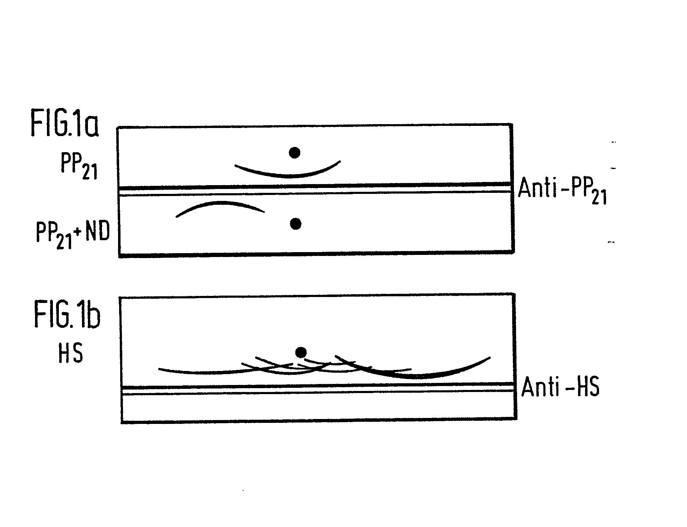

Figur 1a zeigt die immunologische Reaktion von PP$_{21}$ (ohne oder mit dem Enzym Neuraminidase (ND) aus Vibrio comma behandelt) mit einem spezifischen Antiserum vom Kaninchen nach Auftrennung im elektrischen Feld in agarhaltigem Gel. Das unbehandelte native Protein wird im Bereich der $\beta_1$-Globuline gefunden; durch Abspaltung der Neuraminsäure wird das Protein stärker basisch und tritt dann im Bereich der $\gamma$-Globuline auf.

Figur 1b bringt zum Vergleich dazu die Auftrennung der Proteine des Serums, sichtbar gemacht durch deren Immunreaktion mit einem Antiserum vom Kaninchen gegen Humanserum (HS).

Zum immunologischen Nachweis von PP$_{21}$ können auch die Geldiffusionstechnik nach Ouchterlony (vgl. Schultze and Heremans, Molecular Biology of Human Proteins, Vol. 1, p. 134) oder, wenn notwendig, auch empfindlichere Methoden wie Radioimmunoassays oder Enzymimmunoassays herangezogen werden.

Der Nachweis und die Bestimmung von PP$_{21}$ haben diagnostische Bedeutung. PP$_{21}$ ist ein Gewebeprotein, das nur in bestimmten Organen in größerer Konzentration vorkommt. Bei einer Erkrankung dieser Organe kann infolge eines vermehrten Zellzerfalls die Konzentration des

Gewebsproteins $PP_{21}$ im Serum oder in anderen Körperflüssigkeiten der Patienten über die Norm ansteigen. Der Nachweis und die Bestimmung von $PP_{21}$ in Körperflüssigkeiten können daher zur Erkennung von Erkrankungen dieser Organe oder auch als Marker zur Überwachung des Krankheitsverlaufs und zur Kontrolle der Therapie eingesetzt werden.

$PP_{21}$ kann also verwendet werden, um Antiseren herzustellen, die dazu dienen können, $PP_{21}$ nachzuweisen und zu bestimmen.

Die Erfindung wird an nachstehenden Beispielen erläutert:

Beispiel 1

A) Extraktion der Plazenten und Fraktionierung des Extraktes mit einem Acridinderivat und Ammoniumsulfat

1.000 kg tiefgefrorene reife menschliche Plazenten wurden im Schneidmischer zerkleinert und mit 1.000 l einer 4 g/l Kochsalzlösung extrahiert. Der Extrakt ist dann, nach Abtrennung des Geweberückstandes durch Zentrifugation, mit einer 200 ml/l Essigsäurelösung auf pH 6,0 eingestellt und unter Rühren mit 200 l einer 30 g/l Lösung von 2-Ethoxi-6,9-diaminoacridin-Lactat (Hoechst AG) versetzt worden. Der Niederschlag wurde abzentrifugiert und verworfen. Der Überstand wurde mit 10 g/l Betonit A (Fa. Erbslöh & Co., Geisenheim/Rhein) versetzt und durch Zugabe von 2 N NaOH auf pH 7,0 eingestellt und filtriert. Das Filtrat ist unter Rühren langsam mit 300 g/l Ammoniumsulfat versetzt worden; das Plazentaprotein $PP_{21}$ fiel dabei, zusammen mit anderen Proteinen, aus. Der Niederschlag wurde abfiltriert; erhalten

- 11 -

wurden etwa 12 kg einer feuchten Paste, die im nachfolgenden als Fraktion A bezeichnet wird.

B) Gelfiltration an Sephadex G-150

500 g der Fraktion A wurden in etwa 400 ml Wasser
gelöst und gegen einen 0,1 mol/l Tris-HCl-Puffer (pH
8,0), der 1 mol/l NaCl und 1 g/l $NaN_3$ enthielt (Pufferlösung II) dialysiert. Die proteinhaltige Lösung
wurde auf eine mit Sephadex G-150 gefüllte Säule (20
x 100 cm) aufgetragen und gelfiltriert. Zum Eluieren
wurde ein 0,1 mol/l Tris-HCl-Puffer, pH 8, enthaltend 1 mol/l NaCl und 1 g/l $NaN_3$ (Pufferlösung II)
verwendet. Die Eluate sind im Geldiffusionstest nach
Ouchterlony mit einem Anti-$MP_2$-Kaninchenserum auf
$PP_{21}$ getestet worden. Gesammelt wurden die Fraktionen, die das $PP_{21}$ in größerer Menge enthielten (Molekulargewichtsbereich zwischen 40.000 und 100.000).
Anschließend wurden die Proteine durch Zugabe von
300 g/l festem Ammoniumsulfat ausgefällt. Der Niederschlag wurde in Wasser gelöst und gegen Pufferlösung
II dialysiert (Fraktion B).

C) Anreicherung von $PP_{21}$ durch Immunadsorption

1. Herstellung eines Anti-$MP_2$-Kaninchenserums
   Gewinnung von Antikörpern gegen $PP_{21}$

Durch Immunisieren von Kaninchen mit der hochmolekularen Fraktion (Sedimentationskoeffizient
etwa gleich oder größer 20 S) der $MP_2$-Proteine
(deutsche Patentanmeldung 33 34 405) wurden Antiseren gewonnen, die Antikörper gegen $PP_{21}$ enthielten. Die Immunisierung erfolgte unter Verwendung von Aluminiumhydroxid als Adjuvans über einen Zeitraum von 6 Wochen und wurde wie folgt

durchgeführt. Die hochmolekulare MP$_2$-Fraktion wurde in physiologischer Kochsalzlösung gelöst (Konzentration 0,06 mg/3ml) und unter Zusatz von Aluminiumhydroxid zu einer Suspension verrührt. Die Kaninchen erhielten an 5 aufeinander folgenden Tagen je 0,06 mg Protein in 3 ml Suspension/Tier i.v. injiziert. Anschließend folgte eine Pause von 9 Tagen. Dann immunisierte man wieder an 5 aufeinander folgenden Tagen mit der oben angegebenen Menge Antigen, ließ wieder eine Pause von 9 Tagen folgen und injizierte schließlich nochmals an 5 aufeinander folgenden Tagen je 0,06 mg des Antigens. Nach einer erneuten Wartezeit von 7 bis 9 Tagen wurden die Tiere entblutet. Nach dem Gerinnen des Blutes wurde das Serum vom Blutkuchen abgeschleudert und gewonnen.

2. Herstellung des Immunadsorbens
----------------------------------------

300 ml eines Anti-MP$_2$-Serums von Kaninchen wurden gegen einen 0,02 mol/l Phosphatpuffer (pH 7,0) dialysiert und zur Abtrennung der Immunglobuline an DEAE-Cellulose chromatographiert. Die Immunglobuline wandern dabei ungehindert durch die DEAE-Cellulose hindurch, während die übrigen Serumproteine großenteils an die DEAE-Cellulose adsorbiert werden. Die Immunglobulinfraktion im Durchlauf (4,78 g Protein) wurde dann mit 478 g besonders gereinigter Agarose in Kugelform (Sepharose 4 B der Pharmacia, Uppsala, Schweden), die mit 59,9 g Bromcyan aktiviert worden war, umgesetzt und so kovalent an einen Träger gebunden. Das Verfahren ist beispielsweise beschrieben von Axen el al., Nature 214, 1302 (1967).

Mit Hilfe eines auf diese Weise hergestellten Immunadsorbens konnte das Protein PP$_{21}$ aus dessen

Lösung, insbesondere aus $PP_{21}$-angereicherten Plazentenfraktionen isoliert werden.

3. Durchführung der Immunadsorption
----------------------------------------

Das Immunadsorbens wurde in Pufferlösung II suspendiert, in eine Chromatographiesäule gefüllt (5,5 x 20 cm) und mit Pufferlösung II nachgespült. Dann wurde die halbe Menge der Fraktion B auf die Säule aufgetragen, wobei $PP_{21}$ immunadsorptiv gebunden wurde. Die Säule wurde gründlich mit Puffer II gespült. Anschließend ist das adsorbierte Protein mit etwa 600 ml 6 mol/l Harnstoff-Lösung von der Säule eluiert worden. Die $PP_{21}$-haltigen Eluate wurden gegen Pufferlösung II dialysiert und im Ultrafilter auf etwa 10 ml eingeengt. Ausbeute pro Adsorption etwa 10 mg $PP_{21}$.

Das Adsorbens in der Säule wurde unmittelbar nach der Elution von $PP_{21}$ wieder mit Pufferlösung II gründlich gewaschen; es ist dann erneut zur immunadsorptiven Bindung von $PP_{21}$ eingesetzt worden.

D) Hochreinigung von $PP_{21}$

Das durch Immunadsorption gewonnene Protein war häufig noch durch unspezifisch gebundene Serumproteine und andere Plazentaproteine verunreinigt. Die Abtrennung der Hauptmenge der Serum-Begleitproteine gelang durch Gelfiltration an Ultrogel®AcA 34. Die restlichen Begleitproteine wurden dann durch inverse oder negative Immunadsorption, das heißt mit Hilfe von trägergebundenen Antikörpern gegen die noch als Verunreinigung vorliegenden Proteine, entfernt. Im wesentlichen handelte es sich dabei um Serumimmunglobuline und um Spuren des Plazentagewebeprotein $MP_1$.

Patentansprüche:

1. Protein PP$_{21}$, gekennzeichnet durch

   a) eine elektrophoretische Beweglichkeit im Bereich
      der von $\beta_1$-Globulinen;

   b) einen isoelektrischen Punkt von 4,6 $\pm$ 0,3;

   c) einen Sedimentationskoeffizienten s$_{20,w}$ von 3,2 $\pm$
      0,2 S;

   d) ein in einer Ultrazentrifuge bestimmtes Molekular-
      gewicht von 52.900 $\pm$ 6.200;

   e) einen Extinktionskoeffizienten $E_{1cm}^{1\%}$(280 nm) von
      10,5 $\pm$ 1,0; und

   e) einen Kohlenhydratanteil von 19,2 $\pm$ 5,2 g/100 g
      (Mannose 1,8 $\pm$ 0,4, Galactose 4,3 $\pm$ 1,0, Fucose
      1,3 $\pm$ 0,3, N-Acetyl-Glukosamin 6,5 $\pm$ 2,0, N-Ace-
      tyl-Neuraminsäure 5,3 $\pm$ 1,5, jeweils g/100 g).

2. Verfahren zur Gewinnung oder Anreicherung des PP$_{21}$
   nach Anspruch 1, dadurch gekennzeichnet, daß ein aus
   Organen, die dieses Protein enthalten, vorzugsweise
   Plazenta, Magen, Darm, Blase oder Milz, mit einer
   verdünnten Salz- oder Pufferlösung gewonnener Extrakt
   einer oder mehreren der folgenden Maßnahmen unterworfen wird:

   a) Fällung des Proteins PP$_{21}$ mit Ammoniumsulfat im
      pH-Bereich von 6 bis 8 und bei 30-60% Sättigung;

   b) Abscheidung von Begleitproteinen mit einer wasserlöslichen Acridinbase bei einem pH-Wert von 6 und
      einer Konzentration der Base von 4 g/l oder weni-
      ger oder Fällung des Proteins PP$_{21}$ mit einer
      wasserlöslichen Acridinbase bei einem pH-Wert zwischen 7 und 9 und einer Konzentration der Base von
      4 bis 8 g/l;

   c) Abscheidung von Begleitproteinen durch Zugabe von
      bis zu 200 ml Ethanol/l bei pH 7;

d) präparative Zonenelektrophorese, wobei die Proteinfraktion mit einer Beweglichkeit im Bereich der $\beta_1$-Globuline gewonnen wird;

e) isoelektrische Fokussierung, wobei die Proteine im pH-Bereich von 4,4 - 4,8 gewonnen werden;

f) Gelfiltration oder Ultrafiltration, wobei die Proteine im Molekulargewichtsbereich von 40.000 bis 100.000 gewonnen werden;

g) Adsorption an einem schwach basischen Ionenaustauscher und Elution des Proteins $PP_{21}$;

h) immunadsorptive Anreicherung.

3. Verwendung des Proteins $PP_{21}$ nach Anspruch 1 zur Gewinnung eines Antiserums und zum Aufbau von immunchemischen Methoden zum Nachweis und zur Bestimmung dieses Proteins, zur Erkennung von Erkrankungen, zur Überwachung von Erkrankungen oder zur Kontrolle einer Therapie.

FIG.1a

PP$_{21}$

PP$_{21}$+ND

Anti-PP$_{21}$

FIG.1b

HS

Anti-HS